# EUROPEAN PATENT SPECIFICATION

(11) **EP 1 970 077 B1**
(45) Date of publication and mention of the grant of the patent: **14.10.2009**
(21) Application number: 07005413.5
(22) Date of filing: 16.03.2007
(51) Int. Cl.: A61K 47/36, C08B 37/00

(54) **A biogradable material with nanopores and electric conductivity and the fabricating method thereof**
Bioabbaubares Material mit Nanoporen und elektrischer Leitfähigkeit sowie Herstellungsverfahren dafür
Matériau biodégradable doté de nanopores et conductivité électrique et son procédé de fabrication

(43) Date of publication of application: 17.09.2008
(73) Proprietor: National Chi Nan University, Nantou Hsien 545 (TW)
(72) Inventor: Yang, Chung-Shi, Taichung City 408 (TW); Lo, Leu-Wei, Sindian City,Taipei County 231 (TW); Chen, Pei-Ru, Yonghe City, Taipei County 234 (TW); Tsai, Pi-Ju, Taoyuan City Taoyuan County 330 (TW)
(74) Representative: Gill, Siân Victoria

(56) References cited:
- WO-A-20/04044281
- DE-A1-102004 041 340
- US-A1- 2005 095 599
- US-A1- 2006 019 259

## Description

### Background of the invention

### (a) Field of the Invention

The present invention is related to a biodegradable material, and more particularly, to a biodegradable material with nanopores and electric conductivity and its fabricating method.

### (b) Description of the Prior Art:

A biodegradable material usually contains chemical bond that allows hydrolysis, e.g., ester, amide and urea groups; therefore, the biodegradable material is able to undergo degradable by itself or to be gradually decomposed into smaller molecules subject to the action by biotic factors, e.g., microorganisms, and enzyme before being exerted out of a biomass through kidney filtration or metabolism. Generally biodegradable materials can be classified into two categories of natural and synthetic polymers, and has been widely applied in medical and pharmaceutical fields.

Meanwhile, a porous material relates to one with high performance that contains micropores and nanopores and may be called a molecular screen to provide comprehensive range of application including catalyzing reaction, absorption for separation and substance purification, fuel cells, electronic materials, environmental treatment, biochemical purpose, etc.

In the biochemical application in pharmaceutical filed, the porous material has been frequently used in drug controlled-release technology for the purpose of allowing the drug to be applied on a target at a rate as desired. A drug controlled release system controls the release rate of the drug entering into a human body while reducing fluctuation of the concentration of the drug in the blood to achieve an uniform effect of the drug. Furthermore, since the drug is capable of providing its treatment results for the target, the risk of overdose or waste in the administration of the drug may be reduced. For a certain drug with comparatively stronger toxicity, e.g., drugs used in chemical treatment of malignant tumors, local drug delivery is a must to prevent the other parts of the body from damaging. Meanwhile, characteristics and constructions of certain materials known as intelligent materials having been also widely applied in drug control during recent years change depending on externally chemical or physical stimulations including pH, light, and/or temperature.

So far, the application of characteristics about porous materials in biodegradable materials limited to micrometer grade has not yet reached the nanometer grade. In addition, the biodegradable material with nanopores at the same time possessing of electric conductivity has not yet been resolved to facilitate the sampling and/or screening of charged biomolecules and to be applied to neural regeneration-related studies. To satisfy further demands and results of the porous/electro-conductive materials applied in bio-medical filed, this inventor based on years of research and hands-on experience discloses a biodegradable material with nanopores and electric conductivity and the fabricating method thereof to help realize the expectations as aforesaid.

### Summary of the invention

The primary purpose of the present invention is to provide a biodegradable material with nanopores that simultaneously achieves results of biocompatibility, biodegradability, elasticity, retarded elasticity, and porosity while giving electric conductivity function as applicable.

Another purpose of the present invention is to provide a fabricating method for the biodegradable material with nanopores in a simple process to effectively provide nanopores to the biodegradable material and further give conductivity to the biodegradable material of the present invention.

To achieve the purposes, a biodegradable material with nanopores of the present invention includes a polysaccharide polymer and multiple nanopores provided to the polysaccharide polymer.

A fabricating method for the biodegradable material with nanopores of the present invention includes preparation of a polysaccharide polymer; multiple biodegradable nanoparticles are implanted onto the polysaccharide polymer; and multiple enzymes are used to digest and decompose those biodegradable nanoparticles to form multiple nanopores on the polysaccharide polymer. Subsequently, nano-grade electrically conductive material can be further implanted on the polysaccharide polymer with nanopores as applicable.

Accordingly, the biodegradable material with nanopores of the present invention is essentially comprised of a polysaccharide polymer that can be digested and decomposed by a biotic factor for the material to become biodegradable.

Whereas the polysaccharide polymer is related to a natural material, the material of the invention is biocompatible. The material of the present invention can be an elastic material due to the bonding characteristics among the polysaccharide high polymer.

A fabricating method for a biodegradable material with nanopores of the present invention involves the use of mutual matching characteristics among biological materials and technical means of enzyme to provide multiple nanopores to the biological material that executes selected permeability transport on biological particles including peptide.

By implanting nano-grade electrically conductive material on the biodegradable material with nanopores and its fabricating method of the present invention, materials fabricated by using the method of the present invention become conductivity.

### brief description of the drawings

Fig. 1 is a flow chart showing a fabricating method of a biodegradable material with nanopores of a preferred embodiment of the present invention;
Figs. 2a and 2b are SEM photos of a biodegradable material with nanopores of a preferred embodiment of the present invention;
Fig. 3 is a schematic view showing an application of the biodegradable material with nanopores of the preferred embodiment of the present invention;
Figs. 4a and 4b are an analysis chart showing results of osmosis of the biodegradable material with nanopores of the preferred embodiment of the present invention;
Fig. 5 is an SEM photo showing the biodegradable material with nanopores of the preferred embodiment of the present invention is coated with carbon nanotube;
Fig. 6a is a top view of an applied product of the biodegradable material with nanopores of the preferred embodiment of the present invention; and
Fig. 6b is an SEM photo showing a local portion of a finished product of the biodegradable material with nanopores of the preferred embodiment of the present invention.

### Detailed description of the preferred embodiments

A biodegradable material with nanopores of the present invention includes a polysaccharide polymer and multiple nanopores on the polysaccharide polymer.

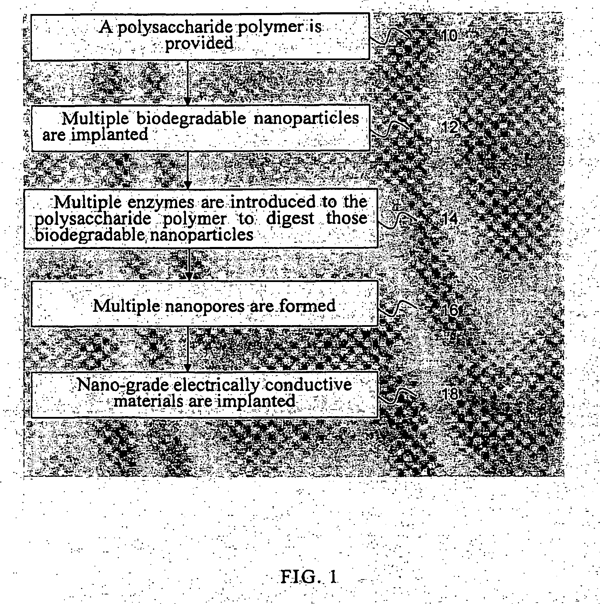

Referring to Fig. 1, a flow chart of a fabricating method for a biodegradable material with nanopores is illustrated. As shown in Fig. 1, a polysaccharide polymer is provided (Step 10), and multiple biodegradable nanoparticles, e.g., gelatin nanoparticles, are implanted on the polysaccharide polymer (Step 12). Wherein, regulation for a desired size of the nanopore becomes feasible by selecting a proper size of the biodegradable nanoparticle. Multiple enzymes, e.g. collagenase, are introduced to the polysaccharide polymer to digest and decompose those biodegradable nanoparticles (Step 14); followed by the formation of multiple nanopores on the polysaccharide polymer (Step 16). In a subsequent step, the polysaccharide polymer with nanopores is implanted with a group of nano-grade electrically conductive material (Step 18); wherein the electrically conductive material can be a carbon nanotube group, nano conductive carbon black group or a combination of both for the material of the present invention to become conductivity.

The polysaccharide polymer may be provided in gels, beads, fibers, colloids, films, nets, or any combination of them to form polysaccharide high molecules of the polysaccharide high polymer such as a group of chitin, chitosan, chitooligo-saccarides comprised of low molecular oligosaccarides including chitobios up to chithexose for example, or any combination of these groups.

The polysaccharide polymer with nanopores can be applied in a micro-dialysis probe or a micro-dialysis probe adapted with optical fibers.

By incorporating optical fibers and the micro-dialysis probe to the biodegradable material with nanopores of the present invention, a material fabricated using the method of the present invention gives biocompatibility, biodegradability, elasticity, retarded elasticity, conductivity, and a molecule detection system allowing regulation of porosity of the material at the same time for the material to be applied in animals; accordingly, an optical fiber and microanalysis detection system using the biodegradable material with nanopores can be used to detect the molecule regulation mechanism of organisms; or the detection system is applied to perform reactions in the organisms.

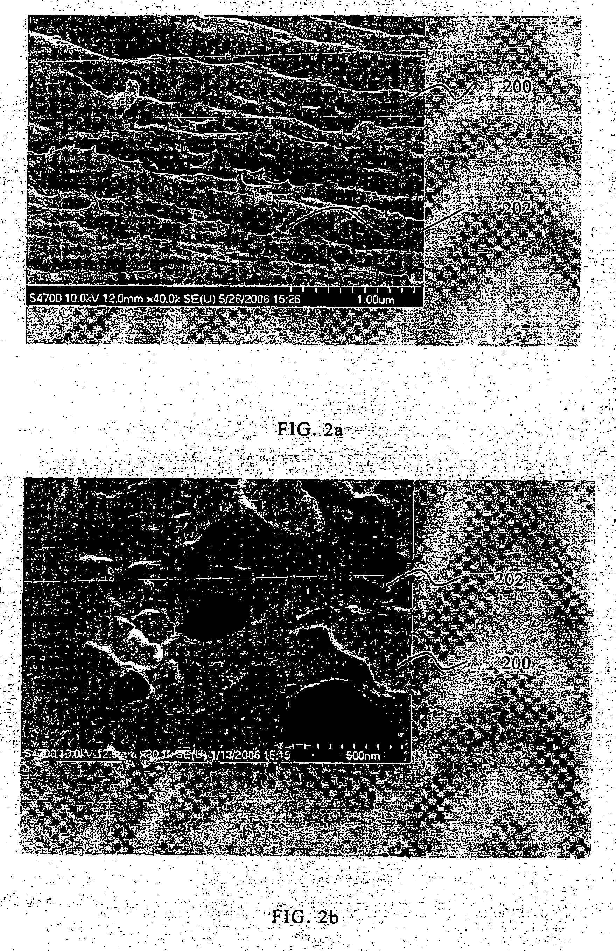

Now referring to SEM photos of a biodegradable material with nanopores according to a preferred embodiment of the present invention given in Figs. 2a and 2b, a collagenase of a 0.2% concentration is used to digest multiple 30~60nm gelatin nanoparticles scattered around a chitosan 200 and then an SEM (scanning electron microscope) is used to observe those nanopores formed on the chitosan 200. The fabricating method of the present invention allows the chitosan 200 to effectively form multiple nanopores 202 as illustrated in Fig. 2a; and those nanopores 202 on the chitosan 200 provides excellent permeability as illustrated in Fig. 2b.

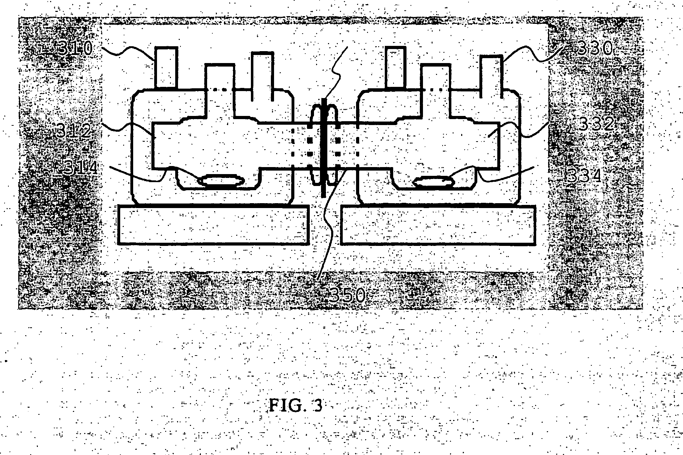

As illustrated in Fig. 3, an application diagram of a biodegradable material with nanopores is shown. Two installations 310 and 330 respectively contain two different solutions 312 and 332. Solution 312 contains receptor cell 314 and solution 332 contains donor cell 334. Both installations 310 and 320 is connected through each other by means of a catheter 350 and a chitosan film 300 with nanopores fabricated by employing the method of the present invention separates both solutions 312 and 332 from each other. Whereas, the chitosan film 300 is provided with multiple nanopores, its porosity permits selective permeability transport among bioparticles including those of peptide.

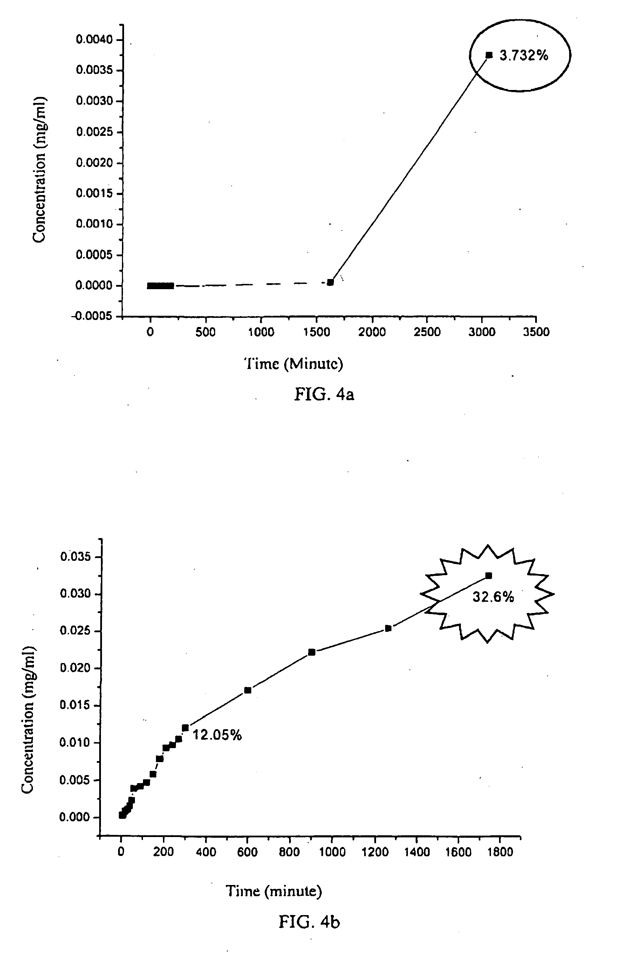

As illustrated in Figs. 4a and 4b, osmosis results of a biodegradable material with nanopores according to an embodiment of a present invention are illustrated. As shown in Fig. 4a, the osmosis result of a concentration of 3.782% can be achieved at 3000-minute for 40kD dextran fluorescein. As shown in Fig. 4b, the osmosis result of a concentration of 32.6% can be achieved at 1700-minute for 3kD dextran fluorescein.

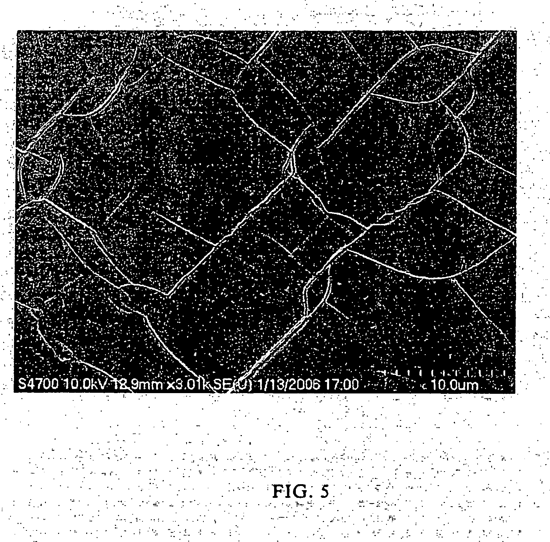

As illustrated in Fig. 5, a SEM photo of carbon nanotubes implanted in a biodegradable material with nanopores according to an embodiment of the present invention is shown. The carbon nanotube (CNT) provides excellent dispersion effects on the biodegradable material with nanopores of the present invention. In a resistance test, the biodegradable material with nanopores of the present invention gives a resistance of 5MΩ.

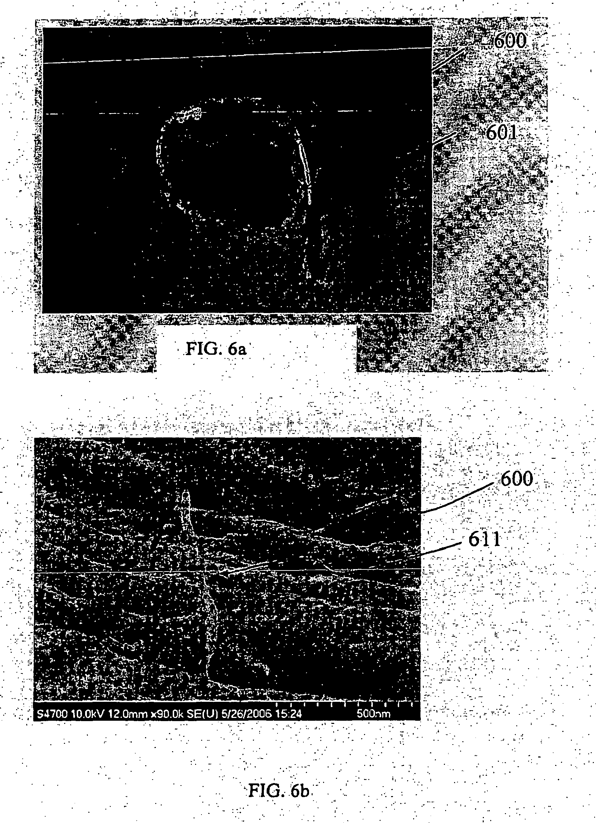

As illustrated in Fig. 6a, a top view of a biodegradable material with nanopores applied to finished goods according to an embodiment of the present invention is shown. As illustrated in Fig. 6b, a SEM photo of a biodegradable material with nanopores applied to finished goods according to an embodiment of the present invention is shown. The chitosan film with nanopores in the embodiment is made into a microtube 600 after doping with the CNT. When observed at a local portion 601 of the microtube 600 using the SEM, a conductive nanowire 611 is visible on the material of the present invention as illustrated in Fig. 6b.

Accordingly, the polysaccharide polymer is the primary composition of a biodegradable material with nanopores of the present invention, and it can be digested by a biotic factor thus to become biodegradable. The material of the present invention can be an elastic material due to the bonding characteristics of the polysaccharide polymer.

A method to fabricate a biodegradable material with nanopores of the present invention takes advantages of characteristics of mutual matching among biological materials and technical means of enzymology to give porosity to the material for executing selective transport on peptide. Furthermore, by implanting a nano-grade electrically conductive material, the material of the present invention becomes conductivity.

A biodegradable material with nanopores of the present invention can be further applied in a micro-dialysis probe or a micro-dialysis probe adapted with optical fibers for the material of the present invention to simultaneously carry biocompatibility, elasticity, retarded elasticity, porosity, conductivity, and a molecule detection system allowing the regulation of porosity of the material so as to detect molecule regulation mechanism in organisms or perform the biochemical reaction as required.

The polysaccharide polymer of the present invention, e.g., a natural macromolecular substance made from chitosan provides good histocompatibility without causing rejection; is biodegradable, given with biological function, and virtually non-toxic; and its molecular structure permits greater variability, e.g., polymerization length and bonding. It can be usually made in gels, beads, fibers, colloids, and films.

Furthermore, the polysaccharide polymer contains amino and hydroxyl groups to allow easy chemical modification for the fabrication of diversified derivatives. The polysaccharide polymer, e.g., the chitosan, is antibiotic to prevent a wound from being affected by bacteria; therefore, it is an ideal material for the fabrication of synthetic skin, suture, dialysis diaphragm of artificial kidney, or for applications in drug controlled release, e.g., micro-capsule and porous carrier. The chitosan also promotes healing of a wound and inhibits scarring. Therefore the present invention promises a great potential for development.

A biodegradable material with nanopores of the present invention is preferably a multi-purpose natural polysaccharide polymer, e.g., chitin, chitosan, chitooligosaccarides, or any combination of these groups given with characteristics of biocompatibility, biological function, antibiotic, and chelated with metal ions for applications in the fields of agriculture, medicine, edibles, chemical engineering, and environmental protection in the makings of wound dressings, pads, sutures, antiseptic and odor-proof fabrics, health food, weight management product, stationary enzyme carrier, cosmetics, fruit juice clarifying agents, fruit preservatives, and wastewater treatment agents.
The features disclosed in the foregoing description, in the claims and/or in the accompanying drawings may, both separately and in any combination thereof, be material for realising the invention in diverse forms thereof.

## Claims

1. A biodegradable material with nanopores, comprising:
a polysaccharide polymer film; and
multiple nanopores formed in the polysaccharide polymer film.

2. A biodegradable material as claimed in claim 1, wherein the polysaccharide polymer is formed of chitin, chitosan, chitooligosaccharides or any combination thereof.

3. A biodegradable material as claimed in either of the preceding claims, wherein the polysaccharide polymer further includes nano-grade electrically conductive material.

4. A biodegradable material as claimed in claim 3, wherein the nano-grade electrically conductive material is carbon nanotubes, nano conductive carbon black or a combination thereof.

5. A biodegradable material with nanopores as claimed in any one of the preceding claims, wherein the film is shaped into a microtube.

6. A micro-dialysis probe comprising the biodegradable material as claimed in any one of the preceding claims.

7. A micro-dialysis probe as claimed in claim 6, wherein the micro-dialysis probe is a micro-dialysis probe adapted with optical fibers.

8. A method of making the biodegradable material as claimed in any one of claims 1 to 5, comprising:
providing a polysaccharide polymer;
implanting multiple biodegradable nanoparticles in the polysaccharide polymer; and
digesting the biodegradable nanoparticles using multiple enzymes to form multiple nanopores in the polysaccharide polymer film.

9. A method as claimed in claim 8, wherein the size of the biodegradable nanoparticles regulates the size of the nanopores in the polysaccharide polymer film.

10. A method as claimed in claim 8 or claim 9, wherein the biodegradable nanoparticles include gelatin nanoparticles.

11. A method as claimed in any one of claims 8 to 10, wherein the enzymes include collagenase.

12. A method as claimed in any one of claims 8 to 11, further comprising the step of shaping the nanoporous polysaccharide polymer film into a microtube.

13. A method as claimed in any one of claims 8 to 12, wherein the polysaccharide polymer is formed of chitin, chitosan, chitooligosaccarides or any combination thereof.

14. A method as claimed in any one of claims 8 to 13, wherein nano-grade electrically conductive material is implanted in the polysaccharide polymer.

15. A method as claimed in claim 14, wherein the nano-grade electrically conductive material is carbon nanotubes, nano conductive carbon black or a combination thereof.

## Patentansprüche

1. Biologisch abbaubares Material mit Nanoporen, das Folgendes umfasst:
eine Polysaccharidpolymerfolie; und
mehrere in der Polysaccharidpolymerfolie gebildete Nanoporen.

2. Biologisch abbaubares Material nach Anspruch 1, wobei das Polysaccharidpolymer aus Chitin, Chitosan, Chitooligosacchariden oder einer beliebigen Kombination davon besteht.

3. Biologisch abbaubares Material nach einem der vorherigen Ansprüche, wobei das Polysaccharidpolymer ferner elektrisch leitendes Material in Nano-Qualität beinhaltet.

4. Biologisch abbaubares Material nach Anspruch 3, wobei das elektrisch leitende Material in Nano-Qualität Kohlenstoffnanoröhrchen, nanoleitenden Ruß oder eine Kombination davon umfasst.

5. Biologisch abbaubares Material mit Nanoporen nach einem der vorherigen Ansprüche, wobei die Folie zu einem Mikroröhrchen geformt ist.

6. Mikrodialysesonde, die das biologisch abbaubare Material nach einem der vorherigen Ansprüche umfasst.

7. Mikrodialysesonde nach Anspruch 6, wobei die Mikrodialysesonde eine Mikrodialysesonde ist, die mit optischen Fasern adaptiert ist.

8. Verfahren zur Herstellung des biologisch abbaubaren Materials nach einem der Ansprüche 1 bis 5, das die folgenden Schritte beinhaltet:
Bereitstellen eines Polysaccharidpolymers,
Implantieren mehrerer biologisch abbaubarer Nanopartikel in das Polysaccharidpolymer; und
Verdauen der biologisch abbaubaren Nanopartikel mit mehreren Enzymen, um mehrere Nanoporen in der Polysaccharidpolymerfolie zu bilden.

9. Verfahren nach Anspruch 8, wobei die Größe der biologisch abbaubaren Nanopartikel die Größe der Nanoporen in der Polysaccharidpolymerfolie reguliert.

10. Verfahren nach Anspruch 8 oder Anspruch 9, wobei die biologisch abbaubaren Nanopartikel Gelatine-Nanopartikel beinhalten.

11. Verfahren nach einem der Ansprüche 8 bis 10, wobei die Enzyme Kollagenase beinhalten.

12. Verfahren nach einem der Ansprüche 8 bis 11, das ferner den Schritt des Gestaltens der nanoporösen Polysaccharidpolymerfolie zu einem Mikroröhrchen beinhaltet.

13. Verfahren nach einem der Ansprüche 8 bis 12, wobei das Polysaccharidpolymer aus Chitin, Chitosan, Chitooligosacchariden oder einer beliebigen Kombination davon gebildet wird.

14. Verfahren nach einem der Ansprüche 8 bis 13, wobei elektrisch leitendes Material in Nano-Qualität in das Polysaccharidpolymer implantiert wird.

15. Verfahren nach Anspruch 14, wobei das elektrisch leitende Material in Nano-Qualität Kohlenstoffnanoröhrchen, nanoleitenden Ruß oder eine Kombination davon umfasst.

## Revendications

1. Matériau biodégradable avec des nanopores, comprenant :
un film de polymère polysaccharide ; et
des nanopores multiples formés dans le film de polymère polysaccharide.

2. Matériau biodégradable selon la revendication 1, dans lequel le polymère polysaccharide est formé de chitine, de chitosan, de chitooligosaccharides, ou de toute combinaison de ceux-ci.

3. Matériau biodégradable selon l'une quelconque des revendications précédentes, dans lequel le polymère polysaccharide comprend en outre un matériau nano-grade conducteur de l'électricité.

4. Matériau biodégradable selon la revendication 3, dans lequel le matériau nano-grade conducteur de l'électricité est constitué de nanotubes de carbone, de noir de carbone nanoconducteur, ou d'une combinaison de ceux-ci.

5. Matériau biodégradable avec des nanopores selon l'une quelconque des revendications précédentes, dans lequel le film est formé en un microtube.

6. Sonde de micro-dialyse constituée du matériau biodégradable selon l'une quelconque des revendications précédentes.

7. Sonde de micro-dialyse selon la revendication 6, ladite sonde de micro-dialyse étant une sonde de micro-dialyse adaptée avec des fibres optiques.

8. Procédé de fabrication du matériau biodégradable selon l'une quelconque des revendications 1 à 5, comprenant les opérations consistant à :
fournir un polymère polysaccharide ;
implanter des nanoparticules biodégradables multiples dans le polymère polysaccharide ;et
digérer les nanoparticules biodégradables en utilisant des enzymes multiples pour former des nanopores multiples dans le film de polymère polysaccharide.

9. Procédé selon la revendication 8, dans lequel la taille des nanoparticules biodégradables régule la taille des nanopores dans le film de polymère polysaccharide.

10. Procédé selon la revendication 8 ou la revendication 9, dans lequel les nanoparticules biodégradables comprennent des nanoparticules de gélatine.

11. Procédé selon l'une quelconque des revendications 8 à 10, dans lequel les enzymes comprennent la collagénase.

12. Procédé selon l'une quelconque des revendications 8 à 11, comprenant en outre l'étape consistant à former le film de polymère polysaccharide en un microtube.

13. Procédé selon l'une quelconque des revendications 8 à 12, dans lequel le polymère polysaccharide est formé de chitine, de chitosan, de chitooligosaccharides ou de toute combinaison de ceux-ci.

14. Procédé selon l'une quelconque des revendications 8 à 13, dans lequel le matériau nano-grade conducteur de l'électricité est implanté dans le polymère polysaccharide.

15. Procédé selon la revendication 14, dans lequel le matériau nano-grade conducteur de l'électricité est constitué de nanotubes de carbone, de noir de carbone nanoconducteur, ou d'une combinaison de ceux-ci.
